(19) Europäisches Patentamt
European Patent Office
Office européen des brevets

(11) **EP 4 324 459 A1**

(12) **EUROPEAN PATENT APPLICATION**
published in accordance with Art. 153(4) EPC


(43) Date of publication:
**21.02.2024 Bulletin 2024/08**

(21) Application number: **22788491.3**

(22) Date of filing: **15.04.2022**

(51) International Patent Classification (IPC):
***A61K 9/20*** (2006.01) ***A61K 31/4155*** (2006.01)

(52) Cooperative Patent Classification (CPC):
**A61K 9/20; A61K 31/4155**

(86) International application number:
**PCT/KR2022/005451**

(87) International publication number:
**WO 2022/220636 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.04.2021 KR 20210049613**



(71) Applicant: **LG Chem, Ltd.**
**Seoul 07336 (KR)**

(72) Inventors:
- **YOO, Seok Cheol**
  **Daejeon 34122 (KR)**
- **JANG, Joomyung**
  **Daejeon 34122 (KR)**
- **SEO, Jin A**
  **Daejeon 34122 (KR)**
- **LIM, Dohyung**
  **Daejeon 34122 (KR)**

(74) Representative: **Hoffmann Eitle**
**Patent- und Rechtsanwälte PartmbB**
**Arabellastraße 30**
**81925 München (DE)**


(54) **ORAL FORMULATION CONTAINING 1-(3-CYANO-1-ISOPROPYL-INDOL-5-YL)PYRAZOLE-4-CARBOXYLIC ACID**


(57) The present invention relates to a composite formulation for oral administration comprising an active pharmaceutical ingredient (API) selected from 1-(3-cyano-1-isopropyl-indol-5-yl)pyrazol-4-carboxylic acid or a pharmaceutically acceptable salt thereof, wherein the API has a particle size of granules corresponding to 90% of the maximum particle size in the cumulative particle size distribution (D(0.9)) of 80 um or more and 300 µm or less.

The composite formulation for oral administration according to the present invention has a low friability and an increased dissolution rate even if a high content of API is included, by regulating the particle size of the API to a certain range.


【Fig. 2】

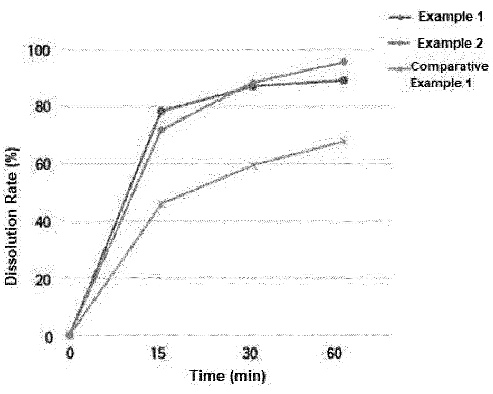


EP 4 324 459 A1

## Description

[Technical Field]

This application claims the benefit of priority based on Korean Patent Application No. 10-2021-0049613 filed on April 16, 2021, the entire contents of which are incorporated herein as part of the present specification.

The present invention relates to a composite formulation for oral administration having excellent physical properties while comprising 1-(3-cyano-1-isopropyl-indol-5-yl)pyrazol-4-carboxylic acid) in a high content.

[Background Art]

Xanthine oxidase is an enzyme that converts hypoxanthine to xanthine and also converts the formed xanthine to uric acid, and the substance inhibiting the activity of xanthine oxidase may effectively treat diseases related to uric acid accumulation, such as hyperuricemia, gout, heart failure, and cardiovascular disease.

On the other hand, with respect to substances that inhibit the activity of xanthine oxidase, Korean Patent No. 10-1751325 (Patent Literature 1) provides 1-(3-cyano-1-isopropyl-indol-5-yl)pyrazol-4-carboxylic acid (Formula 1 below) and a method of preparing the compound; and Korean Patent No. 10-1424013 (Patent Literature 2) provides various types of crystal forms obtained using various solvents and a method of preparing the same.

[Formula 1]

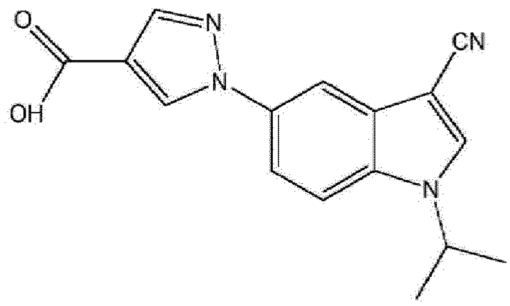

However, there has been no report on a composite formulation for oral administration comprising 1-(3-cyano-1-isopropyl-indol-5-yl)pyrazol-4-carboxylic acid of Formula 1 above or a pharmaceutically acceptable salt thereof as an active pharmaceutical ingredient (API), and in particular, there has been no report on a composite formulation for oral administration comprising the API in a high content.

A composite formulation comprising a high content of API cannot generally be said to have excellent basic physical properties such as hardness and tableting properties, and thus, in order to overcome these disadvantages, it is common to use wet granulation or dry granulation, or to use a method of increasing the amount of excipients to decrease the API ratio.

However, there are problems in that not only may the manufacturing time and production cost increase by proceeding with the granulation process for improving the fluidity of the high content composite formulation, but also the production cost is increased and the patient's acceptability is lowered by increasing the amount of excipients, so increasing the total weight and volume of the composite formulation.

In addition, in the composite formulation for oral administration, when it is provided in the form of an oral tablet in consideration of ease of administration, production cost, etc., bioavailability is very important. An important factor affecting the bioavailability is the dissolution process of the drug *in vivo,* which may be indirectly confirmed by the dissolution rate through a dissolution test in a laboratory.

However, since the high content composite formulation has no choice but to have a lower content excipient compared to the low content composite formulation, the physical properties are generally lowered.

Therefore, there is a need to develop a composite formulation for oral administration having excellent physical properties and excellent dissolution rate, while comprising a high content of 1-(3-cyano-1-isopropyl-indol-5-yl)pyrazol-4-carboxylic acid or a pharmaceutically acceptable salt thereof as an API.

[Prior Art Documents]

[Patent Documents]

(Patent Document 1) 1. Korean Patent No. 10-1751325 (June 21, 2017) titled "novel compounds effective as xanthine oxidase inhibitors, method for preparing the same, and pharmaceutical composition containing the same"
(Patent Literature 2) 2. Korean Patent No. 10-1424013 (July 22, 2014) titled "1-(3-cyano-1-isopropyl-indol-5-yl)pyrazol-4-Carboxylic acid crystalline form and the producing method thereof"

[Disclosure]

[Technical Problem]

It is an object of the present invention to derive a composite formulation for oral administration having excellent physical properties and excellent dissolution rate, while comprising an excess of 1-(3-cyano-1-isopropyl-indol-5-yl)pyrazol-4-carboxylic acid or a pharmaceutically acceptable salt thereof as an API.

[Technical Solution]

The present invention provides relates to a composite formulation for oral administration having excellent physical properties by comprising an API selected from 1-(3-cyano-1-isopropyl-indol-5-yl)pyrazol-4-carboxylic acid or a pharmaceutically acceptable salt thereof, particularly an API having a certain particle size range, and a method of preparing the composite formulation for oral administration.

The API included in the composite formulation for oral administration of the present invention has a particle size of granules corresponding to 90% of the maximum particle size in the cumulative particle size distribution (D(0.9)) of 80 um or more and 300 um or less, or 120 um or more and 270 um or less.

The composite formulation for oral administration of the present invention uses 900 ml of pH 6.8 buffer as a dissolution medium, and when using a paddle with a rotation speed of 50 rpm, the dissolution rate at 15 minutes is 70% or more, and the dissolution rate at 30 minutes is 85% or more, and the dissolution rate at 60 minutes is 89% or more.

The API included in the composite formulation for oral administration of the present invention has a content of 30 to 50% by weight based on the total weight of the composite formulation.

The API in the composite formulation for oral administration of the present invention has a content of 95 to 105%.

The composite formulation for oral administration of the present invention is used for the treatment or prevention of xanthine oxidase-related diseases selected from the group consisting of hyperuricemia, gout, heart failure, cardiovascular disease, hypertension, diabetes, kidney disease, inflammation and joint disease, and inflammatory bowel disease.

[Advantageous Effects]

The composite formulation for oral administration comprising 1-(3-cyano-1-isopropyl-indol-5-yl)pyrazol-4-carboxylic acid or a pharmaceutically acceptable salt thereof according to the present invention as an API may prevent problems that may occur during the manufacturing process due to excellent physical properties even if the high API content is included, by adjusting the particle size of API to a certain range.

In addition, since the composite formulation for oral administration according to the present invention comprises a high content of 1-(3-cyano-1-isopropyl-indol-5-yl)pyrazol-4-carboxylic acid or a pharmaceutically acceptable salt thereof as an API, it is economical, and also increased bioavailability may be expected due to the high dissolution rate.

[Description of Drawings]

Fig. 1 is a result of analyzing the friability (%) of uncoated tablets according to the particle size distribution of API.
Fig. 2 is a result of analyzing the dissolution rate (%) of uncoated tablets according to the particle size distribution of API.

[Best Mode]

Hereinafter, the present invention will be described in more detail.

Unless defined otherwise, all technical terms used in the present invention have the same meaning as commonly understood by those skilled in the art in the relevant field of the present invention. In addition, preferred methods or samples are described in the present specification, but similar or equivalent ones are included in the scope of the present invention. The contents of all publications described by reference in the present specification are incorporated in the present specification by reference in their entirety.

As a result of continuous research in various ways to increase the content of API and to maintain or increase the physical properties in the composite formulation for oral administration comprising 1-(3-cyano-1-isopropyl-indol-5-yl)pyrazol-4-carboxylic acid or a pharmaceutically acceptable salt thereof as the API, the present inventors have developed a composite formulation for oral administration having excellent physical properties and excellent dissolution rate when the particle size of API was adjusted to a certain range.

In this case, as the particle size of API is adjusted to a certain range, physical properties such as flowability are maintained, and when formulated into a tablet (uncoated tablet), it has excellent physical properties such as hardness and friability, so that it is economical, and also there is no need to increase the content of a separate excipient to increase physical properties even if a high content API is included, and the tablet has advantages of having an excellent content and uniformity and having a high dissolution rate.

Therefore, the present invention provides a composite formulation for oral administration having excellent physical properties and a high dissolution rate, by adjusting the particle size of an API selected from 1-(3-cyano-1-isopropyl-indol-5-yl)pyrazol-4-carboxylic acid or a pharmaceutically acceptable salt thereof to a certain range.

The "pharmaceutically acceptable salt" in the present invention refers to a salt form of a compound that does not cause serious irritation to the organism to which the compound is administered and does not impair the biological activity and physical properties of the compound. 1-(3-Cyano-1-isopropyl-indol-5-yl)pyrazol-4-carboxylic acid, which is an API included in the composite formulation for oral administration of the present invention, may be converted into a salt thereof by a conventional method.

In composite formulations for oral administration such as tablets or granular capsules, it is common to try to minimize the size per unit dosage form for easy swallowing even if a high content of API is included, but it is common knowledge of those of ordinary skill in the art that when the API is included in a high content, physical properties such as fluidity and tableting properties are generally impaired, and also bioavailability represented by the dissolution rate may be affected.

In fact, for a composite formulation for oral administration comprising 1-(3-cyano-1-isopropyl-indol-5-yl)pyrazol-4-carboxylic acid as an API, the acceptable content of API in the composite formulation for oral administration (especially tablets) with pharmaceutically acceptable physical properties is only about 30%, and when the content is higher than the value, there is a problem in that physical properties such as tableting properties are rapidly deteriorated.

In the present invention, various studies were conducted on a composite formulation for oral administration having excellent or good bioavailability represented by physical stability and dissolution rate while comprising an API selected from 1-(3-cyano-1-isopropyl-indol-5-yl)pyrazol-4-carboxylic acid or a pharmaceutically acceptable salt thereof in a high content.

The particle size distribution of API is a factor affecting compressibility during the tableting process and is known as one of the important characteristics in the manufacturing process. In addition, the particle size distribution of the API is a factor affecting solubility and is an important characteristic that affects the dissolution of the finished product, and in order to secure a desired dissolution pattern in the finished product, the particle size distribution of the API was adjusted in the present invention.

As a result, by adjusting the particle size distribution of the API, a composite formulation for oral administration having an excellent physical stability and also an excellent dissolution rate even if a high content of API is included has been developed.

One aspect of the present invention provides a composite formulation for oral administration i) comprising 1-(3-cyano-1-isopropyl-indol-5-yl)pyrazol-4-carboxylic acid or a pharmaceutically acceptable salt thereof as API, and ii) wherein the API has a particle size (D(0.9)) of 80 um or more and 300 um or less.

In the present invention, the API may have a particle size of granules corresponding to 90% of the maximum particle size in the cumulative particle size distribution (D(0.9)) of 80 um or more and 300 μm or less, 90 μm or more and 300 um or less, 100 um or more and 300 um or less, 110 um or more and 300 um or less, 120 um or more and 300 um or less, 130 um or more and 300 um or less, 140 um or more and 300 um or less, 150 um or more and 300 um or less, 80 um or more and 270 um or less, 90 um or more and 270 um or less, 100 um or more and 270 um or less, 110 um or more and 270 um or less, 120 um or more and 270 um or less, 130 um or more and 270 um or less, 140 um or more and 270 um or less, 150 um or more and 270 um or less, 80 um or more and 250 um or less, 90 um or more and 250 um or less, 100 um or more and 250 um or less, 110 um or more and 250 um or less, 120 um or more and 250 um

or less, 130 um or more and 250 um or less, 140 um or more and 250 um or less, 150 um or more and 250 μm or less, 80 um or more and 230 μm or less, 90 um or more and 230 μm or less, 100 um or more and 230 μm or less, 110 um or more and 230 μm or less, 120 um or more and 230 μm or less, 130 um or more and 230 μm or less, 140 um or more and 230 μm or less, or 150 um or more and 230 um or less.

In addition, the API may have a particle size of granules corresponding to 50% of the maximum particle size in the cumulative particle size distribution (D(0.5)) of 30 um or more and 150 μm or less, 40 um or more and 150 μm or less, 50 um or more and 150 μm or less, 60 um or more and 150 μm or less, 70 um or more and 150 μm or less, 80 um or more and 150 μm or less, 30 um or more and 140 μm or less, 40 um or more and 140 μm or less, 50 um or more and 140 μm or less, 60 um or more and 140 μm or less, 70 um or more and 140 μm or less, 80 um or more and 140 μm or less, 30 um or more and 130 μm or less, 40 um or more and 130 μm or less, 50 um or more and 130 μm or less, 60 um or more and 130 μm or less, 70 um or more and 130 μm or less, or 80 um or more and 130 μm or less.

In addition, the API may have a particle size of granules corresponding to 10% of the maximum particle size in the cumulative particle size distribution (D(0.1)) of 10 um or more and 90 μm or less, 20 μm or more and 90 μm or less, 30 μm or more and 90 μm or less, 40 μm or more and 90 μm or less, 50 μm or more and 90 μm or less, 10 μm or more and 80 μm or less, 20 μm or more and 80 μm or less, 30 μm or more and 80 μm or less, 40 μm or more and 80 μm or less, or 50 μm or more and 80 μm or less.

The composite formulation for oral administration of the present invention has excellent hardness and low friability because the API has D(0.9) of 80 μm or more and 300 μm or less. As a result, in manufacturing a composite formulation for oral administration (tablet) comprising the particle size-adjusted API of the present invention, there is an advantage in that the productivity of the composite formulation for oral administration may be increased by lowering the possibility of broken and lost tablet during the handling and coating process of the uncoated tablet.

The composite formulation for oral administration of the present invention has an excellent content and uniformity of 95% or more and also has excellent disintegration time.

The composite formulation for oral administration of the present invention uses 900 ml of pH 6.8 buffer as a dissolution medium, and when using a paddle with a rotation speed of 50 rpm, the dissolution rate at 15 minutes is 70% or more, and the dissolution rate at 30 minutes is 85% or more, and the dissolution rate at 60 minutes is 89% or more, thereby showing an excellent dissolution rate compared to a composite formulation for oral administration comprising an API having D(0.9) of 325 μm or more.

In addition, the composite formulation for oral administration of the present invention uses 900 ml of pH 6.8 buffer as a dissolution medium, and when using a paddle with a rotation speed of 50 rpm, the dissolution rate at 60 minutes is 89% or more.

Therefore, since the composite formulation for oral administration of the present invention has an excellent dissolution rate, the bioavailability may also be expected to be excellent by reflecting this.

The composite formulation for oral administration in the present invention further comprises one or more excipients selected from pharmaceutically acceptable diluents, disintegrants, glidants, lubricants, and the like, as excipients.

As excipients such as the diluents, disintegrants, glidants, and lubricants, any of those known to be commonly used in the art may be used. The diluent may be used in an amount of 30 to 50% by weight, 40 to 50% by weight, or 45 to 50% by weight, based on the total weight of the composite formulation for oral administration. The disintegrant may be used in an amount ranging from 1 to 10% by weight or from 1 to 5% by weight, based on the total weight of the composite formulation for oral administration. The glidant may be used in an amount ranging from 0.1 to 5% by weight, 0.2 to 3% by weight, or 0.3 to 2% by weight, based on the total weight of the composite formulation for oral administration. The lubricant may be used in an amount ranging from 0.1 to 10% by weight, 0.3 to 5% by weight, or 0.5 to 4% by weight, based on the total weight of the composite formulation for oral administration.

For example, the diluent may be selected from the group consisting of, but is not limited to, microcrystalline cellulose (MCC), lactose monohydrate, lactose anhydride, lactose, starch, mannitol, carboxymethylcellulose, sorbitol, and combinations thereof. The disintegrant may be selected from the group consisting of, but is not limited to, low-substituted hydroxypropyl cellulose, crospovidone, croscarmellose sodium, sodium starch glycolate, F-melt, and combinations thereof. The glidant may be selected from the group consisting of, but is not limited to, talc, silicon dioxide, and mixtures thereof. The lubricant may be selected from the group consisting of, but is not limited to, magnesium stearate, silicon dioxide, talc, light anhydrous silicic acid, sodium stearyl fumarate, and combinations thereof.

The composite formulation for oral administration may be administered once a day, and may be taken daily.

The content of the API included in the composite formulation for oral administration is 30 to 50% by weight, 35 to 50% by weight, 40 to 50% by weight, 45 to 50% by weight, 30 to 45% by weight, 35 to 45% by weight, 40 to 45% by weight, 30 to 40% by weight, or 35 to 40% by weight, based on the total weight of the composite formulation for oral administration.

The API may be included in an amount of 50 to 500 mg, 50 to 400 mg, 50 to 300 mg, 50 to 200 mg, 50 to 100 mg, 100 to 500 mg, 100 to 400 mg, 100 to 300 mg, 100 to 200 mg, 200 to 500 mg, 200 to 400 mg, 200 to 300 mg, 300 to 500 mg, or 300 to 400 mg per unit dosage form.

The API may be included in an amount of, for example, 50 mg, 100 mg, 150 mg, 200 mg, 300 mg, 400 mg, or 455 mg per unit dosage form.

The content of the API included in the composite formulation of the present invention may be 95% to 105%, 96% to 105%, 97% to 105%, 98% to 105%, 99% to 105%, 100% to 105%, 95% to 100 %, 96% to 100%, 97% to 100%, 98% to 100%, or 99% to 100%. In addition, the API content may be 95% or more, 96% or more, 97% or more, 98% or more, or 99% or more, and may be 105% or less, 104% or less, 103% or less, 102% or less, 101% or less, or 100% or less.

The composite formulation for oral administration of the present invention may be prepared by any method known in the art, by using an active pharmaceutical ingredient (API) selected from 1-(3-cyano-1-isopropyl-indol-5-yl)pyrazol-4-carboxylic acid or a pharmaceutically acceptable salt thereof having the particle size distribution of D(0.9) previously mentioned.

The present invention provides a pharmaceutical composition for treating or preventing human xanthine oxidase-related diseases using a composite formulation for oral administration comprising the 1-(3-cyano-1-isopropyl-indol-5-yl)pyrazol-4-carboxylic acid or a pharmaceutically acceptable salt thereof as an API and glidants.

The present invention provides a method for treating or preventing human xanthine oxidase-related diseases using a composite formulation for oral administration comprising the 1-(3-cyano-1-isopropyl-indol-5-yl)pyrazol-4-carboxylic acid or a pharmaceutically acceptable salt thereof as an API and glidants.

The present invention provides the use for the manufacture of a pharmaceutical composition for treating or preventing human xanthine oxidase-related diseases, comprising the 1-(3-cyano-1-isopropyl-indol-5-yl)pyrazol-4-carboxylic acid or a pharmaceutically acceptable salt thereof as an API.

The "human xanthine oxidase-related disease" of the present invention refers to a disease that may be treated or prevented by inhibiting human xanthine oxidase, and include, for example, hyperuricemia, gout, heart failure, cardiovascular disease, hypertension, diabetes, diabetes-related complications, kidney disease, joint disease, inflammatory bowel disease, and the like, but is not limited to the above-mentioned diseases. Examples of the diabetes-related complications include hyperlipidemia, arteriosclerosis, obesity, hypertension, retinopathy, renal failure, and the like.

The "treating" means stopping or delaying the progression of a disease when used in a subject showing the onset of symptoms, and the "preventing" means stopping or delaying the onset of symptoms when used in a subject who does not show the onset of symptoms but is at high risk thereof.

Unless otherwise indicated, all numbers used in the specification and claims, whether recited or not, are to be understood as being modifiable by the term "about" in all instances. It is also to be understood that the precise numbers used in the specification and claims form additional embodiments of the present disclosure. Efforts have been made to ensure the accuracy of the numerical values disclosed in the examples. However, all measured values may inherently contain certain error values generated from the standard deviations measured in their respective measurement techniques.

Various evaluations in examples and comparative examples were performed as follows.

[Particle size analysis]

The particle size distribution of the API used in examples and comparative examples was measured by a dry method using a laser diffraction particle size analyzer. In this case, D(0.1), D(0.5) and D(0.9) refer to particle diameters of particles corresponding to 10%, 50% and 90% of the total number of particles, respectively, when the measured particles are arranged in order from particles with small particle diameter to particles with large particle diameter.

**[Hardness and friability analysis]**

Hardness and friability are used as indices that may predict broken and lost tablet and the like in handling and coating fixation of tablets (uncoated tablets) after tableting.

For the friability test, tablets are taken in an amount close to about 5 g, the powder attached to the tablets is removed, the mass is precisely measured, and then the tablets are placed in the drum of the friability tester. After the drum is rotated 100 times, the tablets are taken out, the powder attached to the tablets is removed as in the beginning, and then the mass is precisely measured.

$$\text{Friability (\%)} = (\text{mass before test} - \text{mass after test})/(\text{mass before test}) \times 100$$

In the case of the hardness test, 1 tablet is put into a tablet hardness tester and the hardness is measured, and after repeating 10 tablets in this way, its average hardness is used.

**[Disintegration time analysis]**

Disintegration time is an index that affects dissolution and absorption in the body after ingestion of the composite formulation.

After sufficiently filling the disintegration tester with purified water, the temperature is adjusted to 37±2°C. 4 tablets are placed in each glass tube of the disintegration tester, the operation is performed in a prescribed manner, the time at which the tablets completely disintegrate and disappear is measured, and its average value is used.

**[Content analysis]**

A content test was conducted to evaluate the content of the formulated composite formulation. Content is an important quality characteristic of the final product, and the average API content of uncoated tablets was measured.

**<Analytical Conditions>**

Preparation of mobile phase: acetonitrile (500 ml) + purified water (500 ml) + TFA (1 ml)
Preparation of diluent: methanol (900 ml) + purified water (100 ml)
Preparation of standard solution and test solution: After the standard and sample are completely dissolved in the diluent, the analysis is performed according to the UPLC analysis method below.
Column: Waters CSH C18 (2.1 mm I.D. X 100 mm L, Particle size 1.7 um
Column temperature: 40°C
Mobile phase: acetonitrile/$H_2O$/TFA = 500/500/1 (v/v/v)
Flow rate: 0.35 ml/min
Detection: 258 nm uv
Sample amount: 1 μl
Analysis time: 6 minutes

**[Dissolution rate analysis]**

The uncoated tablets of the following examples and comparative examples were tested for dissolution according to the dissolution testing method of the Korean Pharmacopoeia (Tenth Edition). The dissolution method was performed using a paddle method with a stirring speed of 50 rpm and a dissolution temperature of 37±0.5°C. The dissolution medium is 900 ml of pH 6.8 phosphate buffer.

Analysis conditions: The solution obtained in the dissolution test was filtered through a 0.45 um membrane filter, and the concentration of 1-(3-cyano-1-isopropyl-indol-5-yl)pyrazol-4-carboxylic acid as API was analyzed using the UPLC method.

**<Analytical Conditions>**

The analysis conditions are the same as in the content analysis method.

**[Examples and Comparative Examples]**

The tablets of examples and comparative examples were prepared as composite formulations (uncoated tablets) using the components in the corresponding contents as shown in Table 1 below.

1-(3-cyano-1-isopropyl-indol-5-yl)pyrazol-4-carboxylic acid (API) having each particle size distribution and a glidant are mixed and then milled to prepare a first mixture. Thereafter, a diluent, a disintegrant and a lubricant, which are components not included in the first mixture, are mixed and then milled to prepare a second mixture.

After the first mixture and the second mixture are mixed, tableting is performed under conditions of prepressure: 5.0 kN and main pressure: 14 to 15 kN using a rotary tablet press (Modul P, GEA, Belgium) to prepare uncoated tablets.

PRUV® used as a lubricant is a trade name, and its ingredient is sodium stearyl fumarate.

[Table 1]

| Classification | Ingredient | Example 1 | | Example 2 | | Comparative Example 1 | |
|---|---|---|---|---|---|---|---|
| | | Content (mg/T) | Content Ratio (%) | Content (mg/T) | Content Ratio (%) | Content (mg/T) | Content Ratio (%) |
| API | | 100.0 | 45.5 | 100.0 | 45.5 | 100.0 | 45.5 |
| D (0.1) | | 14 | | 70 | | 118 | |
| D(0.5) | | 47 | | 126 | | 203 | |
| D(0.9) | | 101 | | 231 | | 325 | |
| Diluent | MCC 102 (Microcrys talline cellulose) | 104.8 | 47.6 | 104.8 | 47.6 | 104.8 | 47.6 |
| Disintegrant | Crospovido ne | 9.7 | 4.4 | 9.7 | 4.4 | 9.7 | 4.4 |
| Glidant | Colloidal silicon dioxide ($SiO_2$) | 1.1 | 0.5 | 1.1 | 0.5 | 1.1 | 0.5 |
| Lubricant | PRUV® | 4.4 | 2.0 | 4.4 | 2.0 | 4.4 | 2.0 |
| Total weight of tablet (mg) | | 220.0 | 100.0 | 220.0 | 100.0 | 220.0 | 100.0 |

**Test Example 1. Characterization analysis of composite formulation according to particle size distribution**

In the composite formulation for oral administration comprising 1-(3-cyano-1-isopropyl-indol-5-yl)pyrazol-4-carboxylic acid as an API, the physical properties according to the particle size distribution of the API were analyzed through the analysis method described above.

Specifically, the physical properties (hardness, friability, disintegration time, content, and dissolution rate) of the composite formulation (uncoated tablet) prepared using the components in the corresponding content as shown in Table 1 were analyzed, and the results are summarized in shown in Table 2 below and Figures 1 and 2.

The hardness of the uncoated tablet increased as its particle size decreased, whereas the friability increased as its particle size increased, on the contrary. Therefore, when D(0.9) is 325 um or more (Comparative Example 1), considering the hardness and friability of the uncoated tablet, it was not suitable for formulating into tablets (Figure 1).

There was no difference in average content according to particle distribution. However, in the case of dissolution rate, the tablet of Comparative Example 1 showed that all of the average dissolution rates at 15 minutes, 30 minutes and 60 minutes (46.1%, 59.4%, 67.8%) were lower than those of Examples 1 and 2.

In addition, although both Examples 1 and 2 show a relatively good dissolution rate, Examples 1 and 2 show a slight difference for each time period: In the case of the average dissolution rate at 15 minutes, it is 78.5% in Example 1 and 71.8% in Example 2, so the tablet of Example 1 with small particles shows a rather high average dissolution rate, but in the case of the average dissolution rates at 30 minutes and 60 minutes, Example 2 shows a higher average dissolution rate.

[Table 2]

| Comments | | Example 1 | Example 2 | Comparative Example 1 |
|---|---|---|---|---|
| Hardness (kP) | | 10.2 | 7.3 | 6.2 |
| Friability (%) | | 0.07 | 0.05 | 0.12 |
| Disintegration time (s) | | 14 | 10 | 13 |
| Content | Average content (%) | 99.4 | 99.4 | 99.1 |
| Dissolution rate | Average dissolution rate (15 minutes) (%) | 78.5 | 71.8 | 46.1 |
| | Average dissolution rate (30 minutes) (%) | 87.2 | 88.6 | 59.4 |
| | Average dissolution rate (60 minutes) (%) | 89.4 | 95.8 | 67.8 |

So far, the present invention has been described with reference to the preferred embodiments. However, it will be understood by those skilled in the art that various changes and modifications may be made in the present invention without departing from the spirit or scope of the present invention. Accordingly, the disclosed embodiments should be considered as being exemplary and not limiting. The scope of the present invention is defined in the claims rather than the detailed description, and all differences within the equivalent range should be interpreted as being included in the invention.

## Claims

1. A composite formulation for oral administration comprising an active pharmaceutical ingredient (API) selected from 1-(3-cyano-1-isopropyl-indol-5-yl)pyrazol-4-carboxylic acid or a pharmaceutically acceptable salt thereof, wherein the API has a particle size of granules corresponding to 90% of the maximum particle size in the cumulative particle size distribution (D(0.9)) of 80 um or more and 300 μm or less.

2. The composite formulation for oral administration according to claim 1, wherein the API has D(0.9) of 120 um or more and 270 um or less.

3. The composite formulation for oral administration according to claim 1, wherein the composite formulation further comprises one or more excipients selected from diluents, disintegrants, glidants, and lubricants.

4. The composite formulation for oral administration according to claim 1, wherein the API has a content of 30 to 55% by weight based on the total weight of the composite formulation.

5. The composite formulation for oral administration according to claim 4, wherein the API has a content of 40 to 50% by weight based on the total weight of the composite formulation.

6. The composite formulation for oral administration according to claim 1, wherein the API has a content of 50 mg, 100 mg, 200 mg or 300 mg per unit dosage form.

【Fig. 1】

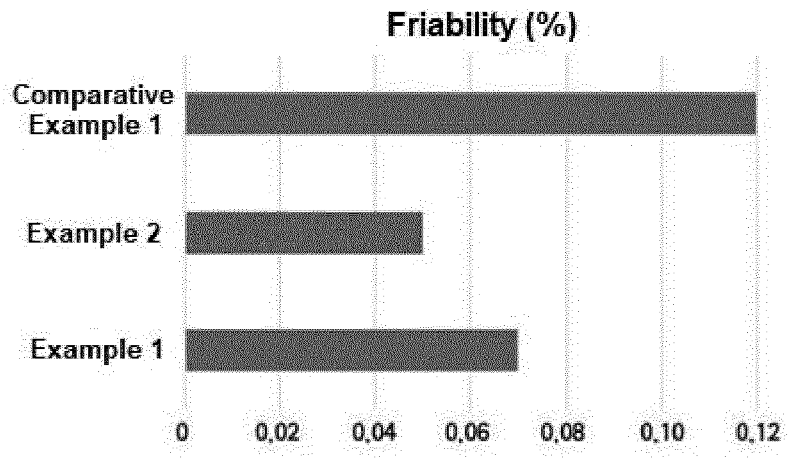
Friability (%)
Comparative Example 1
Example 2
Example 1
0
0,02
0,04
0,06
0,08
0,10
0,12

【Fig. 2】

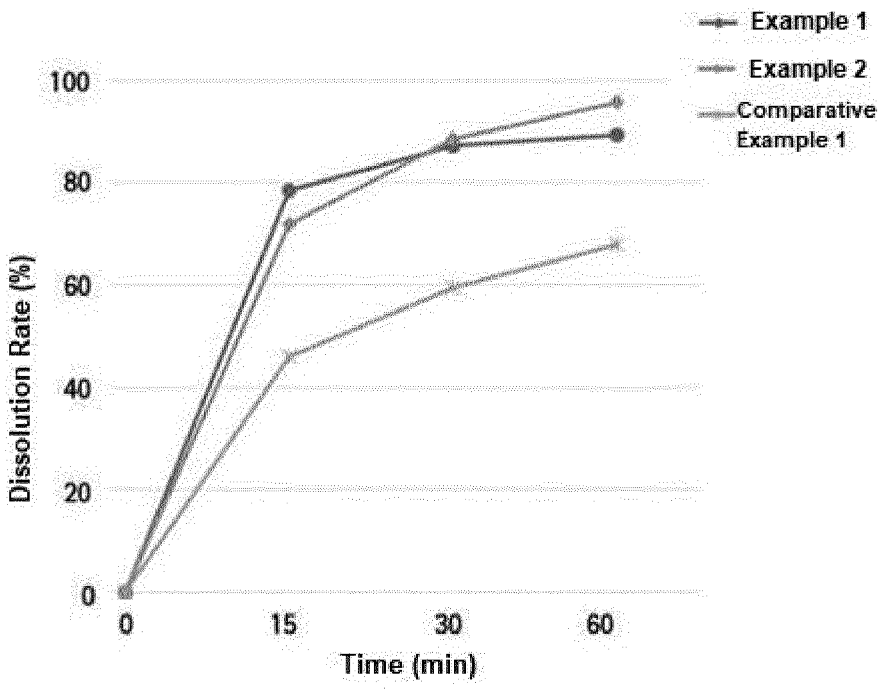
Example 1
Example 2
Comparative Example 1
Dissolution Rate (%)
100
80
60
40
20
0
0
15
30
60
Time (min)

## INTERNATIONAL SEARCH REPORT

International application No.
**PCT/KR2022/005451**

**A. CLASSIFICATION OF SUBJECT MATTER**

**A61K 9/20**(2006.01)i; **A61K 31/4155**(2006.01)i

According to International Patent Classification (IPC) or to both national classification and IPC

**B. FIELDS SEARCHED**

Minimum documentation searched (classification system followed by classification symbols)

A61K 9/20(2006.01); A61K 31/4155(2006.01); A61K 47/36(2006.01); C07D 403/04(2006.01); C07D 413/04(2006.01); C07D 417/04(2006.01); C07D 491/056(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1-(3-시아노-1-아이소프로필-인돌-5-일)피라졸-4-카르복실산(1-(3-cyano-1-isopropyl-indole-5-yl)pyrazole-4-carboxylic acid), 활성제약성분(active pharmaceutical ingredient), 과립(granule), d(0.9), 경구용(oral), 제제(formulation)

**C. DOCUMENTS CONSIDERED TO BE RELEVANT**

| Category* | Citation of document, with indication, where appropriate, of the relevant passages | Relevant to claim No. |
|---|---|---|
| DY | KR 10-2011-0037883 A (LG LIFE SCIENCES LTD.) 13 April 2011 (2011-04-13)<br>See paragraphs [0195], [0198] and [0204]; and claims 1, 9 and 13. | 1-6 |
| Y | KR 10-2010-0012867 A (TEVA PHARMACEUTICAL INDUSTRIES LTD.) 08 February 2010 (2010-02-08)<br>See paragraphs [0033] and [0103]-[0105]; and claims 1, 15, 54 and 55. | 1-6 |
| DY | KR 10-2012-0114174 A (LG LIFE SCIENCES LTD.) 16 October 2012 (2012-10-16)<br>See paragraphs [0022]-[0026]; and claims 1 and 11. | 1-6 |
| A | KR 10-2009-0128488 A (KISSEI PHARMACEUTICAL CO., LTD.) 15 December 2009 (2009-12-15)<br>See entire document. | 1-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 July 2022** | **18 July 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2022/005451**

**C. DOCUMENTS CONSIDERED TO BE RELEVANT**

| Category* | Citation of document, with indication, where appropriate, of the relevant passages | Relevant to claim No. |
|---|---|---|
| A | CN 106008488 A (GUANGDONG HEC PHARMACEUTICAL CO., LTD.) 12 October 2016 (2016-10-12)<br>See entire document. | 1-6 |

Form PCT/ISA/210 (second sheet) (July 2019)

**INTERNATIONAL SEARCH REPORT**
**Information on patent family members**

International application No.
**PCT/KR2022/005451**

| Patent document cited in search report | Publication date (day/month/year) | Patent family member(s) | Publication date (day/month/year) |
|---|---|---|---|
| KR 10-2011-0037883 A | 13 April 2011 | AP 201206191 A0 | 30 April 2012 |
| | | AP 3346 A | 31 July 2015 |
| | | AR 078504 A1 | 09 November 2011 |
| | | AU 2010-304091 A1 | 19 April 2012 |
| | | AU 2010-304091 B2 | 09 June 2016 |
| | | BR 112012007828 A2 | 08 March 2016 |
| | | BR 112012007828 B1 | 12 January 2021 |
| | | BR 112012007828 B8 | 25 May 2021 |
| | | CA 2774133 A1 | 14 April 2011 |
| | | CA 2774133 C | 25 June 2019 |
| | | CL 2012000738 A1 | 14 September 2012 |
| | | CN 102574839 A | 11 July 2012 |
| | | CN 102574839 B | 25 November 2015 |
| | | CO 6430501 A2 | 30 April 2012 |
| | | EA 021025 B1 | 31 March 2015 |
| | | EA 201270520 A1 | 30 October 2012 |
| | | EC SP12011793 A | 30 May 2012 |
| | | EP 2467378 A2 | 27 June 2012 |
| | | EP 2467378 B1 | 27 July 2016 |
| | | ES 2599829 T3 | 03 February 2017 |
| | | HK 1170224 A1 | 22 February 2013 |
| | | IL 218669 A | 31 May 2012 |
| | | IL 218669 B | 31 August 2016 |
| | | JP 2013-507355 A | 04 March 2013 |
| | | JP 5702392 B2 | 15 April 2015 |
| | | MA 33880 B1 | 02 January 2013 |
| | | MX 2012003782 A | 31 August 2012 |
| | | MY 162813 A | 31 July 2017 |
| | | PE 10882012 A1 | 17 August 2012 |
| | | PE 20121088 A1 | 17 August 2012 |
| | | SG 179186 A1 | 30 May 2012 |
| | | TR 201203989 T1 | 21 September 2012 |
| | | TW 201118077 A | 01 June 2011 |
| | | TW I423962 B | 21 January 2014 |
| | | UA 110197 C2 | 10 December 2015 |
| | | US 2012-0184582 A1 | 19 July 2012 |
| | | US 8729273 B2 | 20 May 2014 |
| | | WO 2011-043568 A2 | 14 April 2011 |
| | | WO 2011-043568 A3 | 29 September 2011 |
| | | ZA 201202544 B | 29 December 2012 |
| KR 10-2010-0012867 A | 08 February 2010 | AU 2008-245597 A1 | 06 November 2008 |
| | | BR PI0810814 A2 | 29 October 2014 |
| | | CA 2685184 A1 | 06 November 2008 |
| | | CN 101678114 A | 24 March 2010 |
| | | EP 1985310 A1 | 29 October 2008 |
| | | EP 1985310 B1 | 20 March 2013 |
| | | ES 2405787 T3 | 03 June 2013 |
| | | JP 2010-525082 A | 22 July 2010 |
| | | MX 2009011588 A | 05 November 2009 |
| | | RU 2009141538 A | 27 May 2011 |

Form PCT/ISA/210 (patent family annex) (July 2019)

**INTERNATIONAL SEARCH REPORT**
**Information on patent family members**

International application No.
**PCT/KR2022/005451**

| Patent document cited in search report | Publication date (day/month/year) | Patent family member(s) | Publication date (day/month/year) |
|---|---|---|---|
| | | RU 2456989 C2 | 27 July 2012 |
| | | US 2009-0098211 A1 | 16 April 2009 |
| | | WO 2008-134557 A2 | 06 November 2008 |
| | | WO 2008-134557 A3 | 05 February 2009 |
| KR 10-2012-0114174 A | 16 October 2012 | CN 103459381 A | 18 December 2013 |
| | | CN 103459381 B | 09 December 2015 |
| | | JP 2014-510133 A | 24 April 2014 |
| | | JP 6008937 B2 | 19 October 2016 |
| | | TW 201245182 A | 16 November 2012 |
| | | TW I548630 B | 11 September 2016 |
| | | WO 2012-138147 A2 | 11 October 2012 |
| | | WO 2012-138147 A3 | 29 November 2012 |
| KR 10-2009-0128488 A | 15 December 2009 | AU 2008-239017 A1 | 23 October 2008 |
| | | AU 2008-239017 B2 | 27 September 2012 |
| | | BR PI0810524 A2 | 21 October 2014 |
| | | BR PI0810524 B1 | 10 September 2019 |
| | | BR PI0810524 B8 | 25 May 2021 |
| | | CA 2682391 A1 | 23 October 2008 |
| | | CA 2682391 C | 08 July 2014 |
| | | CN 101679251 A | 24 March 2010 |
| | | CN 101679251 B | 02 October 2013 |
| | | CY 1114647 T1 | 14 December 2016 |
| | | DK 2133332 T3 | 30 September 2013 |
| | | EP 2133332 A1 | 16 December 2009 |
| | | EP 2133332 B1 | 18 September 2013 |
| | | EP 2402314 A1 | 04 January 2012 |
| | | EP 2402314 B1 | 31 July 2013 |
| | | ES 2431815 T3 | 28 November 2013 |
| | | HK 1140197 A1 | 08 October 2010 |
| | | HR P20130900 T1 | 08 November 2013 |
| | | JP 5330989 B2 | 30 October 2013 |
| | | MX 2009010966 A | 02 November 2009 |
| | | MX 337527 B | 09 March 2016 |
| | | PL 2133332 T3 | 28 February 2014 |
| | | PT 2133332 E | 03 October 2013 |
| | | RU 2009141614 A | 20 May 2011 |
| | | RU 2477274 C2 | 10 March 2013 |
| | | SI 2133332 T1 | 28 February 2014 |
| | | US 2010-0056521 A1 | 04 March 2010 |
| | | US 2011-0230454 A1 | 22 September 2011 |
| | | US 2013-0252955 A1 | 26 September 2013 |
| | | US 2014-0179932 A1 | 26 June 2014 |
| | | US 8003647 B2 | 23 August 2011 |
| | | US 8466152 B2 | 18 June 2013 |
| | | US 8829040 B2 | 09 September 2014 |
| | | US 8993616 B2 | 31 March 2015 |
| | | WO 2008-126898 A1 | 22 July 2010 |
| | | WO 2008-126898 A1 | 23 October 2008 |
| CN 106008488 A | 12 October 2016 | CN 106008488 B | 30 October 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210049613
- KR 101751325
- KR 101424013